# EUROPEAN PATENT APPLICATION

(11) **EP 4 353 209 A1**
(43) Date of publication of application: **17.04.2024**
(21) Application number: 21944940.2
(22) Date of filing: 08.11.2021
(51) Int. Cl.: A61F 13/49

(54) **SACRUM-PROTECTING DIAPER**

(30) Priority: 08.06.2021 ES 202100269 U
(71) Applicant: Fundación Profesor Novoa Santos, 15006 A Coruña (ES)
(72) Inventor: FERNÁNDEZ VARELA, José Luis, 15159 Montrove, Oleiros La Coruña (ES)
(74) Representative: ABG Intellectual Property Law, S.L.
(86) International application number: PCT/ES2021/000037
(87) International publication number: WO 2022/258851

(57) **Abstract**

Disclosed is a sacrum-protecting diaper consisting of a diaper specially designed to protect the sacral area of patients with urinary incontinence who must remain in bed for prolonged periods of time.

## Description

### TECHNICAL OBJECT OF THE INVENTION

The present invention describes a sanitary garment to be used by people who, for various reasons, are obliged to remain in bed for long periods of time.

Normally these are elderly people who suffer from urinary incontinence, but they may also be patients with certain ailments who, in addition to suffering from urinary incontinence, must remain in bed for prolonged periods of time and be attended to by healthcare personnel who assist them at times when they require postural changes to avoid bedsores and ulcers.

### TECHNICAL FIELD TO WHICH THE INVENTION RELATES

The invention described concerns the Sector of Daily Living Necessities within the chapter of Health and Protection as regards Medical Sciences and Hygiene, directly affecting the clinical and pharmaceutical industry as regards the manufacture of complements for auxiliary garments for the sick, the elderly and children.

### BACKGROUND OF THE INVENTION

Technology in hospitals and healthcare centers is subject to multiple improvements and advances of all types for the benefit of the patients who come to these centers and also so that doctors, healthcare personnel and professionals, in their functions of caring for the sick, can carry out these tasks with greater precision and efficiency.

The same can be said of the numerous accessories used to enable patients to cope with their sometimes long stays and ailments in the best possible conditions and with maximum comfort.

It is well known to health professionals that patients who spend long periods of time in the supine decubitus position often suffer bedsores, ulcerations, and other damage to the skin on the back side of the body known as the sacral area or simply the sacrum, which is a substantially triangular-shaped bone located at the end of the spine between the fifth lumbar spine vertebra (L5) and the coccyx, popularly known as the tailbone.

This problem is significantly accentuated in patients suffering from urinary incontinence because, even if diapers with a high absorption capacity are used, urine frequently wets the skin in the aforementioned area, causing the lesions mentioned in the previous paragraphs.

To avoid this, there are known prior art documents describing special multi-layer, highly absorbent diapers. A garment registered as "anti-decubitus diaper", protected by patent ES-2242466 A1, which describes a somewhat complex garment as it also covers the protection of the trochanter areas, is also known.

The invention entitled "Urine-detection device with adhesive tape and emitter sensor" protected by the patent WO2019058002A1, with very advanced features, relates to detecting as quickly as possible the moment in which the diaper becomes wet, warning the caregivers through a control center with wireless transmission. In other words, this device prevents the patient from remaining in a wet diaper for a long time, provided that the caregivers react quickly.

In view of the current state of the art, the inventor proposes herein a simple and inexpensive solution that very significantly favors patients who are bedridden for prolonged periods of time who suffer from urinary incontinence. It is a novel solution in relation to which there is no known prior art. With the idea conceived by a Public Home Care practitioner, it is expected to achieve spectacular results in the healing of the sacrum in its initial phase of wound formation and in the prevention of the final formation of bedsores.

### BRIEF DESCRIPTION OF THE INVENTION

As indicated above, the invention relates to a diaper specially designed to protect the sacral area of patients with urinary incontinence who must remain in bed for prolonged periods of time. It has a simple and effective manufacture and represents a breakthrough in the prevention of lesions, wounds and bedsores in the sacrum of bedridden patients. Costs for the manufacturer would be minimal, as it is intended to be made from the same material as that used in the main diaper.

In short, it can be said that it consists of a diaper of the usual type, preferably having a high absorption capacity, complemented with a small diaper part of the same characteristics that is superimposed on the main diaper, duly placed so that it coincides with the sacrum.

The perimeter of the added part is sealed, with its impermeable part being adjacent to the absorbent part of the main diaper and the absorbent part of the added part in contact with the patient's body. Under these conditions, it causes the urine to only soak the main diaper, leaving the sacral area dry and clean, with the advantage that the patient feels more comfortable as the sacral area is in contact with a double-padded diaper surface.

Sacral ulcers are usually caused by moisture, skin maceration and pressure; this method eliminates all three causes.

The inventor proposes various embodiments which are distinguished by the shape of the added part. In a first embodiment, the part is heart-shaped, resembling that of the sacral area, and in others the part has different shapes which can be rectangular, square or any other, preferring those which minimize the cost of manufacturing the diaper of the invention.

The use of this special diaper brings several advantages both to its users and to the healthcare field in general, such as:
- by keeping the protected area dry, it prevents the deterioration of the skin, and therefore the formation of bedsores and ulcerations;
- in cases where wounds already exist, infections are prevented and healing thereof is favored;
- greater comfort for the person by including a double-padded softer area;
- low economic cost;
- less urgency in carrying out postural changes, even if these have to be made in any case.

Studies conducted by the GNEAUPP (National Group for the study and advice on pressure ulcers and chronic wounds), show that more than 90,000 people suffer a pressure ulcer in Spain every day, especially in heels and the sacrum. In hospitals, this is 9 out of every 100 people. The cost of pressure ulcers exceeds 600 million Euros per year and their prevention would cost no more than 1.70 Euros/patient per day, while curing them costs 48 Euros per day, with the industry dedicated to dressings billing 150 million Euros per year. Hygiene and postural changes are fundamental in pressure ulcers. They increase the risk of mortality four-fold and six-fold if the process is complicated by infections.

The gluteal area is the area most prone to develop pressure ulcers, especially the sacrum, representing 50% of all ulcers, while heels represent 30%.

National and international diaper manufacturers have always tried to improve absorption, but they have never considered that with the same diaper they could prevent bedsores in bedridden patients, using the aforementioned method and with a minimum cost.

### BRIEF DESCRIPTION OF THE DRAWINGS

Three figures are included and considered to be sufficient for the proper interpretation of the invention.
Figure 1 schematically depicts the view of the diaper of the invention in which the following elements are indicated:
   1. Protective diaper
   2. Absorbent side
   3. Impermeable side
   4. Complementary part
   5. Sealed edge
Figure 2 depicts another embodiment in which the complementary part is square-shaped. For greater clarity in the arrangement of the layers, a slightly raised tip of the part has been depicted.
Figure 3 depicts a third embodiment in which the complementary part is rectangular-shaped.

### DETAILED DESCRIPTION OF EMBODIMENTS OF THE INVENTION

Disclosed is a sacrum-protecting diaper consisting of a diaper specially designed to protect the sacral area of patients with urinary incontinence who must remain in bed for prolonged periods of time.

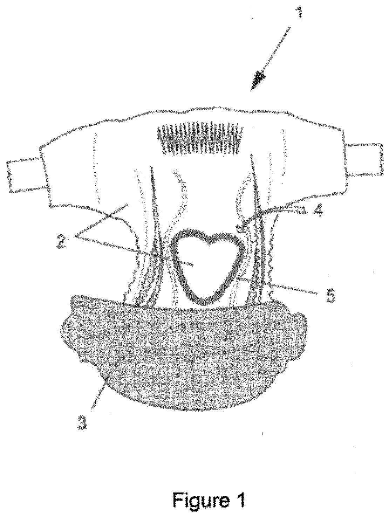

In a first embodiment preferred by the inventor, the sacrum-protecting diaper (1) (Figure 1) is shown as a diaper of the type normally used in healthcare, in particular those consisting of several layers of absorbent material, in which a complementary part (4) made of the same materials as the main diaper that supports it is incorporated.

The complementary part (4) is cutout according to a heart shape that resembles the shape and size of the sacral area of a person, having an absorbent side (2), an impermeable side (3) and a sealed edge (5). The absorbent side (2) of the complementary part (4) is what remains in contact with the patient's body and the impermeable side (3) remains in contact with the absorbent side (2) of the support diaper in such a way that the urine soaks the support diaper but bypasses the complementary part (4), which remains dry.

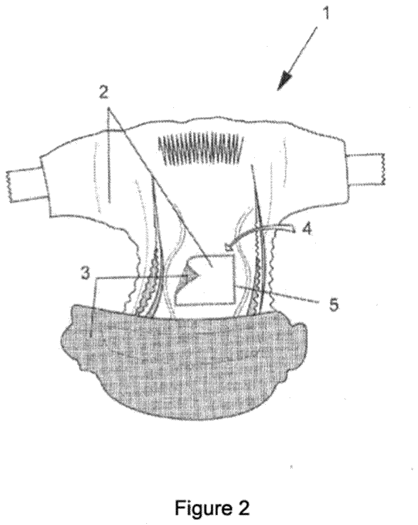

Figure 2 shows the case of a second embodiment in which the complementary part (4) is square-shaped. In this figure, the complementary part (4) has been depicted with one of its tips slightly raised to better show the position of its outer layers, i.e., the absorbent side (2) being visible so that it remains adhered to the body and the impermeable side (3) adjacent to the support diaper.

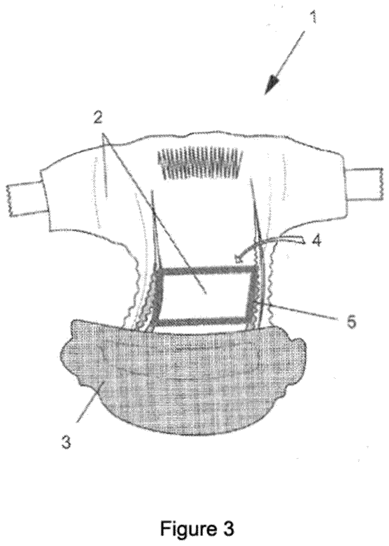

Finally, Figure 3 depicts another one of the possible embodiments where the complementary part (4) is substantially rectangular-shaped.

In all cases, the support diaper and the complementary part (4) have the same structure based on several cellulose layers with a high absorption capacity which, in addition to fulfilling the main objective, provide special protection for the patient who, by keeping the sacral area dry, benefits from the double padding.

The garment of the invention is highly useful and is the first choice of healthcare personnel (doctors, nurses, assistants, caregivers) for application in hospitals, nursing homes, day-care centers and even for bedridden patients at home.

It is not considered necessary to further expand on the subject matter of this description so that a person skilled in the art can understand the scope and advantages derived from the invention, as well as develop and put the object of said invention into practice. However, it should be understood that the invention has been described according to preferred embodiments thereof, and modifications may be considered as long as they do not affect or alter the basis of said invention in any way. In other words, the terms in which this description of the invention has been set forth should always be taken in a broad and non-limiting manner.

## Claims

1. A sacrum-protecting diaper consisting of a diaper specially designed to protect the sacral area of patients with urinary incontinence who must remain in bed for prolonged periods of time, **characterized by** the fact that it is a conventional diaper of the type normally used with several absorbent inner layers and an impermeable outer layer, wherein a complementary part (4) structured in the same way as the support diaper, with an absorbent side (2) and an impermeable side (3), provided with a sealing edge (5), is incorporated, by adhering or by any other method, in an area coinciding with the sacral area.

2. The sacrum-protecting diaper according to claim 1, **characterized in that** the complementary part (4) is heart-shaped.

3. The sacrum-protecting diaper according to claim 1, **characterized in that** the complementary part (4) is square-shaped.

4. The sacrum-protecting diaper according to claim 1, **characterized in that** the complementary part (4) is rectangular-shaped.

5. The sacrum-protecting diaper according to any of the preceding claims, **characterized in that** the impermeable side (3) of the complementary part (4) is placed such that it is in contact with the absorbent side (2) of the support diaper.

## Amended claims

### Amended claims under Art. 19.1 PCT

1. A sacrum-protecting diaper consisting of a diaper specially designed to provide protection to the sacral area against the effects of moisture, pressure and chafing in patients with urinary incontinence who must remain in bed for prolonged periods of time, **characterized by** the fact that it is a conventional diaper of the type normally used with several absorbent inner layers and an impermeable outer layer, wherein a complementary part (4) having the particularity of being sealed along its entire contour, forming an impermeable barrier that prevents in its entirety moisture resulting from incontinence (direct moisture and return moisture in sanitary terms), is incorporated by adhering or by any other method, with the complementary part (4), placed to coincide with the sacral area, having a structure identical to that of the support diaper with a dry absorbent side (2) which acts as a double padding, and an impermeable side (3), being provided with the mentioned sealing edge (5)

2. The sacrum-protecting diaper according to claim 1, **characterized in that** the complementary part (4), which is sealed and, therefore, impermeable to moisture, is heart-shaped.

3. The sacrum-protecting diaper according to claim 1, **characterized in that** the complementary part (4), which is sealed and, therefore, impermeable to moisture, is square-shaped, rectangular-shaped or has any other regular polygon shape.

4. The sacrum-protecting diaper according to any of the preceding claims, **characterized in that** the impermeable side (3) of the complementary part (4) is placed such that it is in contact with the absorbent side (2) of the support diaper.
